# EUROPEAN PATENT SPECIFICATION

(11) **EP 3 057 590 B1**
(45) Date of publication and mention of the grant of the patent: **27.11.2019**
(21) Application number: 14853712.9
(22) Date of filing: 17.10.2014
(51) Int. Cl.: A61K 31/4985, A61K 31/53, A61K 31/5365, A61P 1/16, C07D 487/04, C07D 498/04, C07F 9/6561

(54) **TREATMENT OF HEPATIC FIBROSIS USING AN INHIBITOR OF CBP/CATENIN**
BEHANDLUNG VON LUNGENFIBROSE MIT EINEM CBP-/CATENIN-HEMMER
TRAITEMENT DE LA FIBROSE HÉPATIQUE À L'AIDE D'UN INHIBITEUR DE LA CBP/CATÉNINE

(30) Priority: 18.10.2013 US 201361892606 P
(43) Date of publication of application: 24.08.2016
(73) Proprietor: PRISM BioLab Co., Ltd., Fujisawa-shi Kanagawa 251-0052 (JP)
(72) Inventor: KOUJI, Hiroyuki, Yokohama-shi, Kanagawa 225-0003 (JP); ODAGAMI, Takanao, Toyonaka-shi, Osaka 561-0861 (JP)
(74) Representative: J A Kemp LLP
(86) International application number: PCT/IB2014/002846
(87) International publication number: WO 2015/056104

(56) References cited:
- EP-A1- 2 932 977
- WO-A1-2006/101858
- WO-A1-2009/148192
- WO-A1-2012/115286
- WO-A1-2014/092154
- WO-A2-2011/127164
- JP-A- 2008 533 155
- JP-A- 2011 522 037
- 'WNT ANTAGONISM INHIBITS HEPATIC STELLATE CELL ACTIVATION AND LIVER FIBROSIS' AM J PHYSIOL GASTROINTEST LIVER PHYSIOL vol. 294, 2008, pages G39 - G49, XP009098423
- YOSUKE OSAWA ET AL: "Inhibition of Cyclic Adenosine Monophosphate (cAMP)-response Element-binding Protein (CREB)-binding Protein (CBP)/[beta]-Catenin Reduces Liver Fibrosis in Mice", EBIOMEDICINE, vol. 2, no. 11, 1 November 2015 (2015-11-01), pages 1751-1758, XP055555619, ISSN: 2352-3964, DOI: 10.1016/j.ebiom.2015.10.010

## Description

### BACKGROUND OF THE DISCLOSURE

Hepatitis B is an infectious inflammatory illness of the liver caused by the hepatitis B virus (HBV). The infection is often asymptomatic, but chronic infection can lead to scarring of the liver and ultimately to cirrhosis, which is generally apparent after many years. In some cases, those with cirrhosis will go on to develop liver failure, liver cancer or life-threatening esophageal and gastric varices.

The development of liver fibrosis is a critical event in the natural history of chronic hepatitis B virus infection. Fibrosis is the predominant cause of morbidity and mortality from this disease and is responsible for the development of liver decompensation, hepatocellular carcinoma and death in a percentage of chronically-infected individuals.

Nonalcoholic steatohepatitis or NASH is a common, often "silent" liver disease. It resembles alcoholic liver disease, but occurs in people who drink little or no alcohol. The major feature in NASH is fat in the liver, along with inflammation and damage. Most people with NASH feel well and are not aware that they have a liver problem. Nevertheless, NASH can be severe and can lead to cirrhosis, in which the liver is permanently damaged and scarred and no longer able to work properly. NASH can slowly worsen, causing scarring or "fibrosis" to appear and accumulate in the liver. As fibrosis worsens, cirrhosis develops; the liver becomes scarred, hardened, and unable to function normally.

Liver fibrosis is characterized and defined by the accumulation of fibrous tissue in the liver. Formation of fibrous scar tissue is a normal bodily response to injury, but in fibrosis this healing process goes wrong. When hepatocytes (functional liver cells) are injured due to infection with a virus, heavy alcohol consumption, toxins, trauma, or other factors, the immune system is activated to repair the damage. The injury or death (necrosis) of hepatocytes stimulates inflammatory immune cells to release cytokines, growth factors, and other chemicals. These chemical messengers direct support cells in the liver called hepatic stellate cells to activate and produce collagen, glycoproteins (such as fibronectin), proteoglycans, and other substances. These substances are deposited in the liver, causing the build-up of extracellular matrix (nonfunctional connective tissue). At the same time, the process of breaking down or degrading collagen is impaired. In a healthy liver, the synthesis (fibrogenesis) and breakdown (fibrolysis) of matrix tissue are in balance. Fibrosis occurs when excessive scar tissue builds up faster than it can be broken down and removed from the liver.

Wnt/β-catenin signaling is emerging as a forerunner for its critical roles in many facets of human biology. This signaling pathway has roles in embryogenesis, organogenesis, and maintaining tissue and organ homeostasis, and also in pathological conditions such as cancer and other human disorders such as inflammatory disorders and fibrosis. In liver, it is integral in several physiological events such as development, regeneration, and growth. However, aberrant activation of this pathway is also evident in many different tumors of the liver, and recent studies are beginning to identify its role in additional hepatic pathological conditions. It contributes to liver physiology and pathology by regulating various basic cellular events, including differentiation, proliferation, survival, oxidative stress, morphogenesis, and others.

The role of Wnt/β-catenin signaling is also becoming evident in stellate cells. Although expression of β-catenin in cell-cell contacts of stellate cells is known, nuclear and cytoplasmic β-catenin was reported during liver regeneration more recently. Stellate cell activation is implicated in liver fibrosis in multiple conditions including alcoholic steatohepatitis, non-alcoholic steatohepatitis, and viral hepatitis and as a consequence of metabolic liver diseases. Studies are now beginning to investigate the role of Wnt/β-catenin signaling in stellate cell activation. Recently, DNA microarray analysis of quiescent and activated rat hepatic stellate cells showed upregulation of genes involved in the non-canonical Wnt pathway, but an absence of β-catenin activation. Although this needs to be further characterized, another study examining absence or presence of all Wnt and frizzled genes showed no difference in their overall expression in active or resting stellate and Kupffer cells. However, this study identified the presence of Fzb (sFRP1) only in the activated cells, suggesting modulation of Wnt signaling during stellate or Kupffer cell activation (Thompson, M. and Monga, S., Hepatology 45: 1298-1305, 2007).

Activation of hepatic stellate cells (HSC), a key event in liver fibrosis, is caused by diminished adipogenic transcription. Recent studies have investigated whether Wnt signaling contributes to "antiadipogenic" activation of HSC and liver fibrogenesis (Cheng J. et al., Am. J. Physiol. Gastrointest. Liver Physiol. 294:G39-G49, 2008). In these studies, culture-activated HSC from normal rats and HSC from cholestatic rat livers were examined for expression of Wnt, Frizzled (Fz) receptors, and coreceptors by quantitative PCR. Wnt signaling was assessed by nuclear beta-catenin and T cell factor (TCF) promoter activity. Dickkopf-1 (Dkk-1), a Wnt coreceptor antagonist, was transduced by an adenoviral vector to assess the effects of Wnt antagonism on culture activation of HSC and cholestatic liver fibrosis in mice. Messenger RNA for canonical (Wnt3a and 10b) and noncanonical (Wnt4 and 5a) Wnt genes, Fz-1 and 2, and coreceptors [low-density lipoprotein-receptor-related protein (LRP)6 and Ryk] are increased approximately 3 to 12-fold in culture-activated HSC compared with quiescent HSC. The nuclear beta-catenin level and TCF DNA binding are markedly increased in activated HSC.

TCF promoter activity is stimulated by Wnt1 but inhibited by Chibby, a protein that blocks beta-catenin interaction with TCF, and by Dkk-1. Dkk-1 enhances peroxisome proliferator-activated receptor-gamma (PPAR-gamma)-driven PPAR response element (PPRE) promoter activity, a key adipogenic transcriptional parameter, abrogates agonist-stimulated contraction, and restores HSC quiescence in culture. High expression of Dkk-1 increases apoptosis of cultured HSC. Expression of Wnt and Fz genes is also induced in HSC isolated from experimental cholestatic liver fibrosis, and Dkk-1 expression ameliorates this form of liver fibrosis in mice. Thus, antiadipogenic Wnt signaling in HSC activation, and therapeutic potential of Wnt antagonism for liver fibrosis, has been identified (Cheng J. et al., Am. J. Physiol. Gastrointest. Liver Physiol. 294:G39-G49, 2008).

### BRIEF SUMMARY OF THE DISCLOSURE

This disclosure presents methods of treating hepatic fibrosis resulting from steatohepatitis, including hepatic fibrosis associated with non-alcoholic steatohepatitis (NASH), by administration of an inhibitor of β-catenin signaling selected from 4-(((6S,9S,9aS)-1-(benzylcarbamoyl)-2,9-dimethyl-4,7-dioxo-8-(quinolin-8-ylmethyl)octahydro-1H-pyrazino[2,1-c][1,2,4]triazin-6-yl)methyl)phenyl dihydrogen phosphate, or (6S,9S,9aS)-N-benzyl-6-(4-hydroxybenzyl)-2,9-dimethyl-4,7-dioxo-8-(quinolin-8-ylmethyl)octahydro-1H-pyrazino[2,1-c][1,2,4]triazine-1-carboxamide.

### BRIEF DESCRIPTION OF THE FIGURES

Figure 1. Treatment with Compound A reduces fibrosis in NASH rat model.
Figure 2. Representative photomicrographs of Sirius red stained liver sections.

### DETAILED DESCRIPTION OF THE DISCLOSURE

Non-peptide compounds have been developed which mimic the secondary structure of reverse-turns found in biologically active proteins or peptides. For example, U.S. Pat. No. 5,440,013 and published PCT Applications Nos. WO94/03494, WO01/00210A1, and WO01/16135A2 each disclose conformationally constrained, non-peptidic compounds, which mimic the three-dimensional structure of reverse-turns. In addition, U.S. Pat. No. 5,929,237 and its continuation-in-part U.S. Pat. No. 6,013,458, disclose conformationally constrained compounds which mimic the secondary structure of reverse-turn regions of biologically active peptides and proteins. In relation to reverse-turn mimetics, conformationally constrained compounds have been disclosed which mimic the secondary structure of alpha-helix regions of biologically active peptide and proteins in WO2007/056513 and WO2007/056593.

This disclosure provides compounds, pharmaceutical compositions and methods of treatment for hepatic fibrosis resulting from steatohepatitis. The inventors have determined that inhibiting β-catenin signaling is an effective approach to the treatment of fibrotic liver diseases.

Structures and compounds of the alpha helix mimetic β-catenin inhibitors are disclosed in WO 2010/044485, WO 2010/128685, WO 2009/148192, and US 2011/0092459.

The alpha helix mimetic β-catenin inhibitor compound of the present invention is selected from:
4-(((6S,9S,9aS)-1-(benzylcarbamoyl)-2,9-dimethyl-4,7-dioxo-8-(quinolin-8-ylmethyl)octahydro-1H-pyrazino[2,1-c][1,2,4]triazin-6-yl)methyl)phenyl dihydrogen phosphate (Compound A), or
(6S,9S,9aS)-N-benzyl-6-(4-hydroxybenzyl)-2,9-dimethyl-4,7-dioxo-8-(quinolin-8-ylmethyl)octahydro-1H-pyrazino[2,1-c][1,2,4]triazine-1 -carboxamide.

These compounds are for use in the prevention and/or treatment of hepatic fibrosis resulting from steatohepatitis.

While not wishing to be bound, the effectiveness of these compounds in treating these conditions is based in part on the ability of these compounds to block TCF4/β-catenin transcriptional pathway by inhibiting cyclic AMP response-element binding protein (CBP), thus altering wnt pathway signaling, which has been found to improve outcomes.

A "β-catenin inhibitor" is a substance that can reduce or prevent β-catenin activity. β-catenin activities include translocation to the nucleus, binding with TCF (T cell factor) transcription factors, and coactivating TCF transcription factor-induced transcription of TCF target genes. A "β-catenin inhibitor" can also interfere with the interaction of CBP and β-catenin. Thus, a β-catenin inhibitor inhibits or reduces CBP/β-catenin signaling and activity of the CBP/β-catenin signaling pathway, including reduction of one or more downstream signaling events.

Disclosed herein are certain alpha helix mimetic β-catenin inhibitor compounds for treatment of hepatic fibrosis resulting from steatohepatitis.

As used herein, "hepatic fibrosis" is defined as excessive accumulation of connective or scar tissue within the liver. The accumulation of connective/scar tissue in hepatic fibrosis is excessive compared to connective tissue levels in a normal, healthy liver. This fibrosis is often accompanied by necrosis and/or inflammation of liver tissue. In particular, hepatic stellate cells (HSCs) that store vitamin A in the normal liver are converted into myofibroblasts by acute and chronic liver damage, rapidly proliferate and synthesize an excessive amount of connective tissues through the increase in the synthesis and translocation of an extracellular matrix such as collagen, proteoglycan or hyaluronan, which results in stimulating the progression of liver fibrosis [Friedman et al., Proc. Natl. Acad. Sci. USA., 82: 8681 (1985) Gressner et al., Biochem. Biophys. Res. Commun., 151: 222 (1988) Gressner et al., J. Hepatol., 22: 28 (1995)]. CBP/Catenin signaling can induce the proliferation and development of hepatic stellate cells, and thereby plays a role in inducing the over-production and excess accumulation of an extracellular matrix such as collagen.

Steatohepatitis (also known as fatty liver disease) is a type of liver disease characterized by liver inflammation with concurrent fat accumulation in liver. Deposition of fat in the liver is termed steatosis, and together these constitute fatty liver changes. When not associated with excessive alcohol intake, it is referred to as non-alcoholic steatohepatitis, or NASH, and is the progressive form of non-alcoholic fatty liver disease. Steatohepatitis can progress to cirrhosis, and NASH is now believed to be a frequent cause of unexplained cirrhosis (at least in Western societies). In NASH, fat builds up in the liver and eventually causes scar tissue. This type of hepatitis appears to be associated with diabetes, protein malnutrition, obesity, coronary artery disease, and treatment with corticosteroid medications. The alpha helix mimetic β-catenin inhibitor compounds disclosed herein can treat steatohepatitis.

NASH can be graded on a scoring system that combines several histological features: necrosis, polymorphonuclear infiltrate, Mallory bodies and clarification. This scoring system is also useful for diagnosing hepatitis in general. Each feature is scored from 0 to 2 with a total score ranging from 0 to 8 with a four grades scoring system (Mathurin P, et al. Gastroenterology 110:1847-53 (1996); Bedossa P, et al. Alcohol Clin. Exp. Res. 12:173-8 (1988)): NASH can be graded on a 4 grade scoring system adapted from the Brunt score (Angulo P. N. Engl. J. Med. 346(16):1221-31, 2002): Grade 0: score=0, no NASH; Grade 1: score=1-2, mild/ mild Steatosis: predominantly macrovesicular, involves up to 66% of lobules, Ballooning: occasionally observed; zone 3 hepatocytes, Lobular inflammation: scattered and mild acute inflammation (polymorphonuclear cells) and occasional chronic inflammation (mononuclear cells), Portal inflammation: none or mild; Grade 2: score=3-4, moderate: Steatosis: any degree; usually mixed macrovesicular and microvesicular, Ballooning: obvious and present in zone 3, Lobular inflammation: polymorphonuclear cells may be noted in association with ballooned hepatocytcs, pericellular fibrosis; mild chronic inflammation may be seen, Portal inflammation: mild to moderate; Grade 3: score=5-8, severe Steatosis: typically involves >66% of lobules (panacinar); commonly mixed steatosis, Ballooning: predominantly zone 3; marked Lobular inflammation: scattered acute and chronic inflammation; polymorphonuclear cells may be concentrated in zone 3 areas of ballooning and perisinusoidal fibrosis; Portal inflammation: mild to moderate.

As used herein, "treatment" refers to clinical intervention in an attempt to alter the disease course of the individual or cell being treated, and can be performed during the course of clinical pathology. Therapeutic effects of treatment include without limitation, preventing recurrence of disease, alleviation of symptoms, diminishment of any direct or indirect pathological consequences of the disease, decreasing the rate of disease progression, amelioration or palliation of the disease state, and remission or improved prognosis.

As used herein, the terms "therapeutically effective amount" and "effective amount" are used interchangeably to refer to an amount of a composition of the invention that is sufficient to result in the prevention of the development or onset of hepatic fibrosis, or one or more symptoms thereof, to enhance or improve the effect(s) of another therapy, and/or to ameliorate one or more symptoms of hepatic fibrosis. For a subject suffering from hepatic fibrosis, a preferred therapeutically effective amount is an amount effective to reduce fibrosis and/or improve liver function.

A therapeutically effective amount can be administered to a patient in one or more doses sufficient to palliate, ameliorate, stabilize, reverse or slow the progression of the disease, or otherwise reduce the pathological consequences of the disease, or reduce the symptoms of the disease. The amelioration or reduction need not be permanent, but may be for a period of time ranging from at least one hour, at least one day, or at least one week or more. The effective amount is generally determined by the physician on a case-by-case basis and is within the skill of one in the art. Several factors are typically taken into account when determining an appropriate dosage to achieve an effective amount. These factors include age, sex and weight of the patient, the condition being treated, the severity of the condition, as well as the route of administration, dosage form and regimen and the desired result.

As used herein, the terms "subject" and "patient" are used interchangeably and refer to an animal, preferably a mammal such as a non-primate (e.g., cows, pigs, horses, cats, dogs, rats etc.) and a primate (e.g., monkey and human), and most preferably a human.

The alpha helix mimetic β-catenin inhibitors described herein are useful to prevent or treat disease. Specifically, the disclosure provides for both prophylactic and therapeutic methods of treating a subject at risk of (or susceptible to) hepatic fibrosis resulting from steatohepatitis. Accordingly, the present methods provide for the prevention and/or treatment of said hepatic fibrosis in a subject by administering an effective amount of the alpha helix mimetic β-catenin inhibitors to a subject in need thereof. For example, a subject can be administered the alpha helix mimetic β-catenin inhibitors in an effort to improve one or more of the factors of said hepatic fibrosis condition.

The invention also encompasses methods where the compound is given in combination therapy. That is, the compound can be used in conjunction with, but separately from, other agents useful in treating hepatitis and HBV infection. In these combination methods, the compound will generally be given in a daily dose of 1-100 mg/kg body weight daily in conjunction with other agents. The other agents generally will be given in the amounts used therapeutically. The specific dosing regime, however, will be determined by a physician using sound medical judgment.

Some examples of compounds suitable for compositions and methods involving combination therapy with the inhibitory compounds disclosed herein include, but are not limited to, the following: ICG-001; Omega IFN IFN-.omega. Intarcia Therapeutics; BILN-2061 serine protease inhibitor Boehringer Ingelheim Pharma KG, Ingelheim, Germany; Summetrel antiviral Endo Pharmaceuticals Holdings Inc., Chadds Ford, PA; Roferon A IFN-.alpha.2a F. Hoffmann-La Roche LTD, Basel, Switzerland; Pegasys PEGylated IFN-.alpha.2a F. Hoffmann-La Roche LTD, Basel, Switzerland; Pegasys and Ribavirin PEGylated IFN- F. Hoffmann-La Roche .alpha.2a/ribavirin LTD, Basel, Switzerland; CellCept HCV IgG F. Hoffmann-La Roche immunosuppressant LTD, Basel, Switzerland; Wellferon lymphoblastoid IFN- GlaxoSmithKline plc, .alpha.n1 Uxbridge, UK; Albuferon - .alpha. albumin IFN-.alpha.2b Human Genome Sciences Inc., Rockville, MD; Levovirin ribavirin ICN Pharmaceuticals, Costa Mesa, CA; IDN-6556 caspase inhibitor Idun Pharmaceuticals Inc., San Diego, CA; IP-501 antifibrotic Indevus Pharmaceuticals Inc., Lexington, MA; Actimmune INF-.gamma. InterMune Inc., Brisbane, CA; Infergen A IFN alfacon-1 InterMune Pharmaceuticals Inc., Brisbane, CA; ISIS 14803 antisense ISIS Pharmaceuticals Inc, Carlsbad, CA/Elan Phamaceuticals Inc., New York, NY; JTK-003 RdRp inhibitor Japan Tobacco Inc., Tokyo, Japan; Pegasys and Ceplene PEGylated IFN-.alpha.2a/ Maxim Pharmaceuticals immune modulator Inc., San Diego, CA; Ceplene immune modulator Maxim Pharmaceuticals Inc., San Diego, CA; Civacir HCV IgG Nabi immunosuppressant Biopharmaceuticals Inc., Boca Raton, FL; Intron A and Zadaxin IFN-.alpha.2b/.alpha.1-RegeneRx thymosin Biopharmiceuticals Inc., Bethesda, MD/ SciClone Pharmaceuticals Inc, San Mateo, CA; Levovirin IMPDH inhibitor Ribapharm Inc., Costa Mesa, CA; Viramidine Ribavirin Prodrug Ribapharm Inc., Costa Mesa, CA; Heptazyme ribozyme Ribozyme Pharmaceuticals Inc., Boulder, CO; Intron A IFN-.alpha.2b Schering-Plough Corporation, Kenilworth, NJ; PEG-Intron PEGylated IFN-.alpha.2b Schering-Plough Corporation, Kenilworth, NJ; Rebetron IFN-.alpha.2b/ribavirin Schering-Plough Corporation, Kenilworth, NJ; Ribavirin ribavirin Schering-Plough Corporation, Kenilworth, NJ; PEG-Intron/Ribavirin PEGylated IFN- Schering-Plough .alpha.2b/ribavirin Corporation, Kenilworth, NJ; Zadazim Immune modulator SciClone Pharmaceuticals Inc., San Mateo, CA; Rebif IFN-.beta.1a Serono, Geneva, Switzerland; IFN-.beta. and EMZ701 IFN-.beta. and EMZ701 Transition Therapeutics Inc., Ontario, Canada; Batabulin (T67) .beta.-tubulin inhibitor Tularik Inc., South San Francisco, CA; Merimepodib IMPDH inhibitor Vertex Pharmaceuticals (VX-497) Inc., Cambridge, MA; Telaprevir NS3 serine protease Vertex Pharmaceuticals (VX-950, LY-570310) inhibitor Inc., Cambridge, MA/ Eli Lilly and Co. Inc., Indianapolis, IN; Omniferon natural IFN-.alpha. Viragen Inc., Plantation, FL; XTL-6865 (XTL-002) monoclonal antibody XTL Biopharmaceuticals Ltd., Rehovot, Israel.

Treatment of hepatic fibrosis refers to the administration of a compound or combination described herein to treat a subject suffering from hepatic fibrosis. One outcome of the treatment of hepatic fibrosis is to reduce formation of connective tissue. Another outcome of the treatment of hepatic fibrosis is to reduce inflammation and infiltration of immune cells. Still another outcome of the treatment of hepatic fibrosis is to reduce liver tissue necrosis. Still another outcome of the treatment of hepatic fibrosis is to improve liver function.

The alpha helix mimetic β-catenin inhibitors described herein can be incorporated into pharmaceutical compositions for administration, singly or in combination, to a subject for the treatment or prevention of a disorder described herein. Such compositions typically include the active agent and a pharmaceutically acceptable carrier. As used herein the term "pharmaceutically acceptable carrier" includes saline, solvents, dispersion media, coatings, antibacterial and antifungal agents, isotonic and absorption delaying agents, and the like, compatible with pharmaceutical administration. Supplementary active compounds can also be incorporated into the compositions.

Any suitable route of administration may be employed for providing a mammal, especially a human, with an effective dose of a compound described herein. For example, oral, rectal, topical, parenteral, ocular, pulmonary, nasal, and the like may be employed. Dosage forms include tablets, troches, dispersions, suspensions, solutions, capsules, creams, ointments, aerosols, and the like.

The effective dosage of active ingredient employed may vary depending on the particular compound employed, the mode of administration, the condition being treated and the severity of the condition being treated. Such dosage may be ascertained readily by a person skilled in the art.

When treating or controlling hepatic fibrosis diseases for which compounds described herein are indicated, generally satisfactory results are obtained when the compounds described herein are administered at a daily dosage of from about 0.01 milligram to about 100 milligram per kilogram of animal body weight, preferably given as a single daily dose or in divided doses two to six times a day, or in sustained release form. For most large mammals, the total daily dosage is from about 1.0 milligrams to about 1000 milligrams. In the case of a 70 kg adult human, the total daily dose will generally be from about 1 milligram to about 500 milligrams. For a particularly potent compound, the dosage for an adult human may be as low as 0.1 mg. In some cases, the daily dose may be as high as 1 gram. The dosage regimen may be adjusted within this range or even outside of this range to provide the optimal therapeutic response.

Oral administration will usually be carried out using tablets or capsules. Examples of doses in tablets and capsules are 0.1 mg, 0.25 mg, 0.5 mg, 1 mg, 2 mg, 5 mg, 10 mg, 15 mg, 20 mg, 25 mg, 30 mg, 40 mg, 50 mg, 100 mg, 200 mg, 250 mg, 300 mg, 400 mg, 500 mg, and 750 mg. Other oral forms may also have the same or similar dosages.

Also described herein are pharmaceutical compositions which comprise a compound described herein and a pharmaceutically acceptable carrier. The pharmaceutical compositions described herein comprise a compound described herein or a pharmaceutically acceptable salt as an active ingredient, as well as a pharmaceutically acceptable carrier and optionally other therapeutic ingredients. A pharmaceutical composition may also comprise a prodrug, or a pharmaceutically acceptable salt thereof, if a prodrug is administered.

The compositions can be suitable for oral, rectal, topical, parenteral (including subcutaneous, intramuscular, and intravenous), ocular (ophthalmic), pulmonary (nasal or buccal inhalation), or nasal administration, although the most suitable route in any given case will depend on the nature and severity of the conditions being treated and on the nature of the active ingredient. They may be conveniently presented in unit dosage form and prepared by any of the methods well-known in the art of pharmacy.

In practical use, the compounds described herein can be combined as the active ingredient in intimate admixture with a pharmaceutical carrier according to conventional pharmaceutical compounding techniques. The carrier may take a wide variety of forms depending on the form of preparation desired for administration, e.g., oral or parenteral (including intravenous). In preparing the compositions as oral dosage form, any of the usual pharmaceutical media may be employed, such as, for example, water, glycols, oils, alcohols, flavoring agents, preservatives, coloring agents and the like in the case of oral liquid preparations, such as, for example, suspensions, elixirs and solutions; or carriers such as starches, sugars, microcrystalline cellulose, diluents, granulating agents, lubricants, binders, disintegrating agents and the like in the case of oral solid preparations such as, for example, powders, hard and soft capsules and tablets, with the solid oral preparations being preferred over the liquid preparations.

Because of their ease of administration, tablets and capsules represent the most advantageous oral dosage unit form in which case solid pharmaceutical carriers are employed. If desired, tablets may be coated by standard aqueous or nonaqueous techniques. Such compositions and preparations should contain at least 0.1 percent of active compound. The percentage of active compound in these compositions may, of course, be varied and may conveniently be between about 2 percent to about 60 percent of the weight of the unit. The amount of active compound in such therapeutically useful compositions is such that an effective dosage will be obtained. The active compounds can also be administered intranasally as, for example, liquid drops or spray.

The tablets, pills, capsules, and the like may also contain a binder such as gum tragacanth, acacia, corn starch or gelatin; excipients such as dicalcium phosphate; a disintegrating agent such as corn starch, potato starch, alginic acid; a lubricant such as magnesium stearate; and a sweetening agent such as sucrose, lactose or saccharin. When a dosage unit form is a capsule, it may contain, in addition to materials of the above type, a liquid carrier such as a fatty oil.

Various other materials may be present as coatings or to modify the physical form of the dosage unit. For instance, tablets may be coated with shellac, sugar or both. A syrup or elixir may contain, in addition to the active ingredient, sucrose as a sweetening agent, methyl and propylparabens as preservatives, a dye and a flavoring such as cherry or orange flavor.

Compounds described herein may also be administered parenterally. Solutions or suspensions of these active compounds can be prepared in water suitably mixed with a surfactant or mixture of surfactants such as hydroxypropylcellulose, polysorbate 80, and mono and diglycerides of medium and long chain fatty acids. Dispersions can also be prepared in glycerol, liquid polyethylene glycols and mixtures thereof in oils. Under ordinary conditions of storage and use, these preparations contain a preservative to prevent the growth of microorganisms.

The pharmaceutical forms suitable for injectable use include sterile aqueous solutions or dispersions and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (e.g. glycerol, propylene glycol and liquid polyethylene glycol), suitable mixtures thereof, and vegetable oils.

### EXAMPLES

### Example 1. MATERIALS AND METHODS

### Abbreviations:

- ALT: : Alanine aminotransferase
- α-SMA: : Alpha-smooth muscle actin
- BCA: : Bicinchoninic acid
- cDNA: : Complementary DNA
- HE: : Hematoxylin and eosin
- HFD: : High fat diet
- Hyp: : Hydroxyproline
- MMLV-RT: : Moloney murine leukemia virus reverse transcriptase
- NAFLD: : Non-alcoholic fatty liver disease
- NAS: : NAFLD Activity score
- NASH: : Non-alcoholic steatohepatitis
- NTP: : Nucleotide triphosphate
- Rplp0: Ribosomal protein, large, P0
- RT-PCR: : Reverse transcriptase polymerase chain reaction
- QD: : Once daily
- SD: : Standard deviation
- SPF: : Specific pathogen-free
- TG: : Triglyceride
- TIMP-1: : Tissue inhibitor of metalloproteinase-1

**Animals.** C57BL/6J mice (15-day pregnant female) were obtained from Charles River Laboratories Japan (Kanagawa, Japan). All animals used in this study were housed and cared for in accordance with the Japanese Pharmacological Society Guidelines for Animal Use. The animals were maintained in a SPF facility under controlled conditions of temperature (23 ± 2°C), humidity (45 ± 10%), lighting (12-hour artificial light and dark cycles; light from 8:00 to 20:00) and air exchange. A high pressure (20 ± 4 Pa) was maintained in the experimental room to prevent contamination of the facility. The animals were housed in polycarbonate cages KN-600 (Natsume Seisakusho, Japan) with a maximum of 4 mice per cage. Sterilized PULMASµ (Material Research Center, Japan) was used for bedding and replaced once a week. Sterilized solid HFD was provided *ad libitum,* being placed in the metal lid on top of the cage. Pure water was provided *ad libitum* from a water bottle equipped with a rubber stopper and a sipper tube. Water bottles were replaced once a week, cleaned and sterilized in autoclave and reused. Mice were identified by numbers engraved on earrings. Each cage was labeled with a specific identification code.

**Test substance.** The test substance Compound A, which is the β-catenin inhibitor 4-(((6S,9S,9aS)-1-(benzylcarbamoyl)-2,9-dimethyl-4,7-dioxo-8-(quinolin-8-ylmethyl)octahydro-1H-pyrazino[2,1-c][1,2,4]triazin-6-yl)methyl)phenyl dihydrogen phosphate, and the vehicle (phosphate buffered saline, pH 7.6) were stored at 4°C. Compound A was diluted with the vehicle just prior to administration. Telmisartan (MICARDIS®) was purchased from Boehringer Ingelheim GmbH and was dissolved in pure water.

**Induction of NASH.** NASH was induced in 40 male mice by a single subcutaneous injection of 200 µg streptozotocin (Sigma-Aldrich, USA) 2 days after birth and feeding with high fat diet (HFD, 57 kcal% fat, cat# HFD32, CLEA Japan, Japan) after 4 weeks of age. The mice were randomized into 5 groups of 8 mice at 9 weeks of age. Eight male littermates, fed with normal diet (cat# CE-2, CLEA Japan) without streptozotocin treatment, were used for the Normal group.

**Route of drug administration.** Compound A and the vehicle were administered by continuous subcutaneous infusion via osmotic pumps (ALZET pumps, cat# 1004, DURECT Corporation, USA) at an infusion rate of 0.11 µL/hr (2.64 µL/animal/day). The test substance solution was filled in the osmotic pumps at concentrations determined based on the individual body weight at the start of treatment (day 0). Telmisartan was administered by oral route in a volume of 10 mL/kg body weight.

**Treatment dose.** Compound A was infused continuously at doses of 1 mg/kg/day (low dose) or 3 mg/kg/day (high dose). Telmisartan was administered once daily at a dose of 10 mg/kg body weight.

**Measurement of whole blood and serum biochemistry.** Non-fasting blood glucose was measured in whole blood using Life Check (EIDIA, Japan). For serum biochemistry, blood was collected in polypropylene tubes without anticoagulant and kept at room temperature for 30 minutes, followed by at 4°C for 1 hour. The blood samples were then centrifuged at 1,000 xg for 15 minutes at 4°C. The supernatant was collected and stored at - 80°C until use. Serum ALT and TG levels were measured by FUJI DRI-CHEM 7000 (Fujifilm, Japan).

**Measurement of liver hydroxyproline content.** To quantify liver hydroxyproline content, frozen liver samples (35-45 mg) were processed by an alkaline-acid hydrolysis method as follows: the livers were defatted with 100% acetone, dried in the air, dissolved in 2N NaOH at 65°C, and autoclaved at 121°C for 20 minutes. The lysed samples (400 µL) were acid-hydrolyzed with 400 µL of 6N HCl at 121°C for 20 minutes, and neutralized with 400 µL of 4N NaOH containing 10 mg/mL activated carbon. AC buffer (2.2M acetic acid/0.48M citric acid, 400 µL) was added to the samples, followed by centrifugation to collect the supernatant. To construct a standard curve of hydroxyproline, serial dilutions of trans-4-hydroxy-Lproline (Sigma-Aldrich) were prepared starting at 16 µg/mL. The prepared samples and standards (each 400 µL) were mixed with 400 µL chloramine T solution (Wako Pure Chemical Industries, Japan) and incubated for 25 minutes at room temperature. The samples were then mixed with Ehrlich's solution (400 µL) and heated at 65°C for 20 minutes to develop the color. After samples were cooled on ice and centrifuged to remove precipitates, the optical density of each supernatant was measured at 560 nm. The concentrations of hydroxyproline were calculated from the hydroxyproline standard curve. Protein concentrations of liver samples were determined using a BCA protein assay kit (Thermo Fisher Scientific, USA) and used to normalize the calculated hydroxyproline values. Hydroxyproline levels were expressed as µg per mg total protein.

**Measurement of liver TG content.** Liver total lipid-extracts were obtained from right lobes by Folch's method (Folch J. et al, J. Biol. Chem. 1957; 226 (1): 497). The livers were homogenized in chloroform-methanol (2:1, v/v) and incubated overnight at room temperature. After washing with chloroform-methanol-water (8:4:3, v/v/v), the extracts were evaporated to dryness, and dissolved in isopropanol. Liver TG levels were measured by Triglyceride E-test (Wako Pure Chemical Industries). Extracts were diluted 2-to 4-fold in isopropanol when the TG contents exceed the limit of detection.

**Histopathological analyses.** For HE staining, sections were cut from paraffin blocks of liver tissue prefixed in Bouin's solution and stained with Lillie-Mayer's Hematoxylin (Muto Pure Chemicals, Japan) and eosin solution (Wako Pure Chemical Industries). NAFLD Activity score (NAS) was calculated according to the criteria of Kleiner (Kleiner DE. et al., Hepatology, 2005 (41):1313). To visualize collagen deposition, Bouin's fixed liver sections were stained using picro-Sirius red solution (Waldeck GmbH & Co., Germany). For quantitative analysis, bright field images of Sirius red-stained sections were captured around the central vein using a digital camera (DFC280, Leica, Germany) at 200-fold magnification, and the positive areas in 5 fields/section were measured using ImageJ software (ImageJ, National Institute of Health, USA).

**Quantitative RT-PCR.** Total RNA was extracted from liver samples using RNAiso (Takara Bio, Japan) according to the manufacturer's instructions. One µg of RNA was reverse-transcribed using a reaction mixture containing 4.4 mM MgCl2 (Roche, Switzerland), 40 U RNase inhibitor (Toyobo, Japan), 0.5 mM dNTP (Promega, USA), 6.28 µM random hexamer (Promega), 5 x first strand buffer (Promega), 10 mM dithiothreitol (Invitrogen, USA) and 200 U MMLV-RT (Invitrogen) in a final volume of 20 µL. The reaction was carried out for 1 hour at 37°C, followed by 5 minutes at 99°C. Real-time PCR was performed using real-time PCR DICE and SYBR premix Taq (Takara Bio). To calculate the relative mRNA expression level, the expression of each gene was normalized to that of reference gene 36B4 (gene symbol: Rplp0). Information of PCR-primer sets and the plate layout was described in Table 1.

**Statistical analyses.** Statistical analyses were performed using Bonferroni Multiple Comparison Test on Prism 4 Software (GraphPad Software, USA). P values < 0.05 were considered statistically significant. Results were expressed as mean ± SD.

### Example 2. EXPERIMENTAL DESIGN AND TREATMENT

**Study groups.** A summary of the treatment schedule is provided in Table 1.
**Group 1: Normal.** Eight normal mice were fed normal diet *ad libitum* without any treatment from 9 to 12 weeks of age.
**Group 2: Disease-control.** Eight NASH mice were fed HFD *ad libitum* without any treatment from 9 to 12 weeks of age.
**Group 3: Telmisartan.** Eight NASH mice were orally administered pure water supplemented with Telmisartan at a dose of 10 mg/kg daily from 9 to 12 weeks of age.
**Group 4: Vehicle.** Eight NASH mice were subcutaneously administered the vehicle (phosphate buffered saline) by continuous infusion via osmotic pumps at a rate of 0.11 µL/hr from 9 to 13 weeks of age.
**Group 5: Compound A low dose.** Eight NASH mice were subcutaneously administered the vehicle supplemented with Compound A by continuous infusion via osmotic pumps at a dose of 1 mg/kg/day from 9 to 13 weeks of age.
**Group 6: Compound A high dose.** Eight NASH mice were subcutaneously administered the vehicle supplemented with Compound A by continuous infusion via osmotic pumps at a dose of 3 mg/kg/day from 9 to 13 weeks of age.

**Table 1. Summary of treatment schedule**

| Group | No. mice | Test substance | Dose (mg/kg/day) | Volume (ml/Kg) | Regimens | Sacrifice (Wks of age) |
|---|---|---|---|---|---|---|
| 1 | 8 | - | - | - | - | 12 |
| 2 | 8 | - | - | - | - | 12 |
| 3 | 8 | Telmisartan | 10 | 10 | Oral, QD, 9-12 wks | 12 |
| 4 | 8 | Vehicle | - | * | Continuous infusion via osmotic pumps, 9-13 wks | 13 |
| 5 | 8 | Compound A | 1 | * | Continuous infusion via osmotic pumps, 9-13 wks | 13 |
| 6 | 8 | Compound A | 3 | * | Continuous infusion via osmotic pumps, 9-13 wks | 13 |

| | | | | | | |
|---|---|---|---|---|---|---|
| * Continuous infusion at the rate of 0.11 µl/hr (2.64 µl/animal/day) | | | | | | |

**Animal monitoring and sacrifice.** The viability, clinical signs and behavior were monitored daily. Body weight was recorded daily before drug administration. Mice were observed for significant clinical signs of toxicity, moribundity, and mortality approximately 60 minutes after each administration. The animals were sacrificed by exsanguination through direct cardiac puncture under ether anesthesia (Wako Pure Chemical Industries).

### Example 3. Results

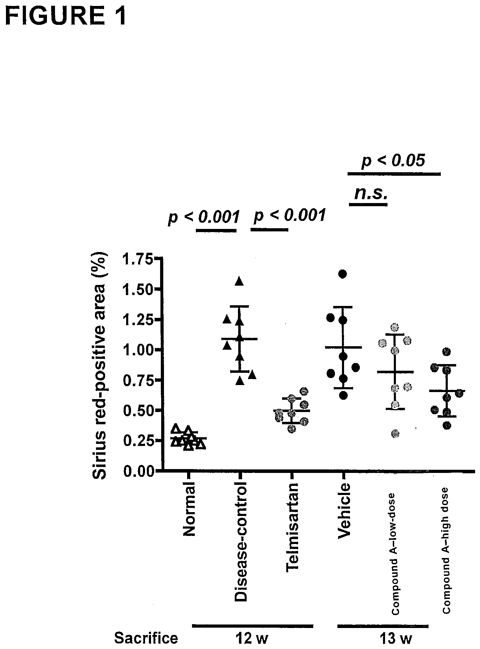

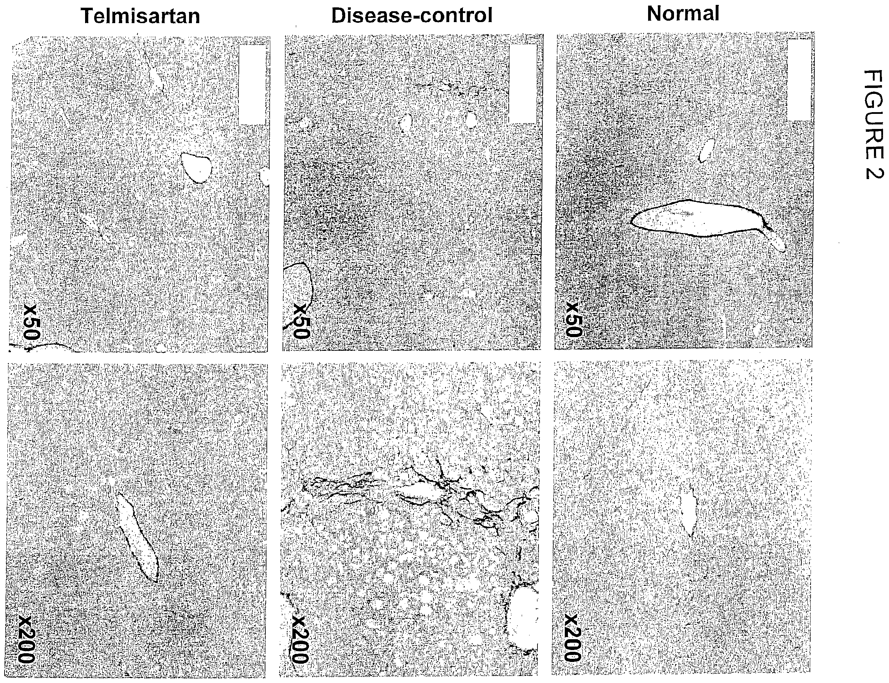

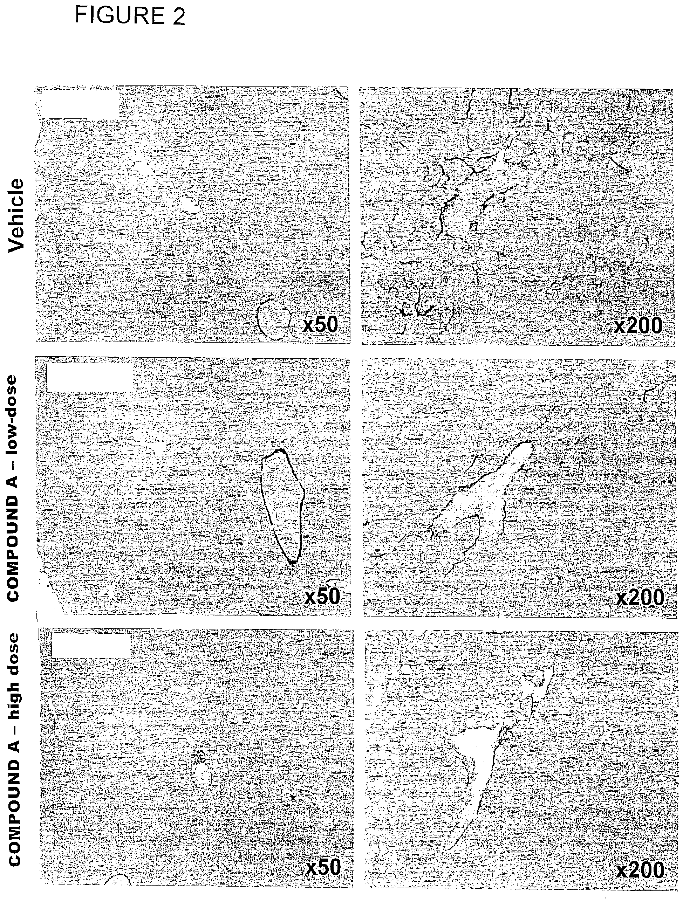

**Sirius red staining.** Liver sections from the Disease-control group showed increased collagen deposition in the pericentral region of the liver lobule compared with the Normal group. The percentage of fibrosis area (Sirius red-positive area) significantly increased in the Disease-control group compared with the Normal group (Normal: 0.27 ± 0.05%, Disease-control: 1.09 ± 0.27%). The fibrosis area significantly decreased in the Telmisartan group compared with the Disease-control group (Telmisartan: 0.50 ± 0.10%). There was no significant difference in the fibrosis area between the Vehicle group and the Compound A low-dose group (Vehicle: 1.02 ± 0.33%, Compound A low-dose: 0.82 ± 0.31%). See **Table 2.** As seen in Figure 1, fibrosis area significantly decreased in the Compound A high-dose group compared with the Vehicle group (Compound A high-dose: 0.67 ± 0.21%). Representative photomicrographs of the Sirius red-stained sections are shown in Figure 2.

**TABLE 2. FIBROSIS AREA**

| | 12 weeks of age | | | 13 weeks of age | | |
|---|---|---|---|---|---|---|
| PARAMETER (mean ± SD) | NORMAL (n+8) | Disease-control (n=8) | Telmisartan (n=8) | Vehicle (n=8) | Compound A low-dose (n=8) | Compound A high dose (n=8) |
| | | | | | | |
| Sirius red-positive area (%) | 0.27 ± 0.05 | 1.09 ± 0.27 | 0.50 ± 0.10 | 1.02 ± 0.33 | 0.82 ± 0.31 | 0.67 ± 0.21 |

**Body weight changes and general condition.** Body weight gradually increased during the treatment period in all except the Telmisartan group. Mean body weight of Disease-control group was lower than that of Normal group throughout the treatment period. Mean body weight of Telmisartan group was significantly lower than that of Disease-control group from day 6 to day 21 (sacrifice) except for day 7. There were no significant differences in mean body weight between the vehicle group and either the Compound A low-dose or high-dose groups during the treatment period. No deterioration in general condition was observed in any of the animals throughout the treatment period.

**Body weight at the day of sacrifice.** Mean body weight at sacrifice was significantly lower in the Disease-control group than in the Normal group (Normal: 26.3 ± 0.8 g, Disease-control: 23.0 ± 1.2 g). The Telmisartan group showed a significant decrease in mean body weight compared with the Disease-control group (Telmisartan: 20.0 ± 1.3 g). There were no significant differences in mean body weight between the Vehicle group and either the Compound A low-dose or high-dose groups (Vehicle: 22.9 ± 2.9 g, Compound A low-dose: 22.6 ± 2.6 g, Compound A high-dose: 23.7 ± 1.1 g).

**Liver weight and liver-to-body weight ratio.** Mean liver weight significantly increased in the Disease-control group compared with the Normal group (Normal: 1128 ± 44 mg, Disease-control: 1867 ± 183 mg). The Telmisartan group showed a significant decrease in mean liver weight compared with the Disease-control group (Telmisartan: 1376 ± 80 mg). There were no significant differences in mean liver weight between the Vehicle group and either the Compound A low-dose or high-dose groups (Vehicle: 1870 ± 418 mg, Compound A low-dose: 1803 ± 219 mg, Compound A high-dose: 2024 ± 159 mg). The liver-to-body weight ratio significantly increased in the Disease-control group compared with the Normal group (Normal: 4.3 ± 0.2%, Disease-control: 8.1 ± 0.6%). The Telmisartan group showed a significant decrease in the liver-to-body weight ratio compared with the Disease-control group (Telmisartan: 6.9 ± 0.7%). There were no significant differences in the liver-to-body weight ratio between the Vehicle group and either the Compound A low-dose or high-dose groups (Vehicle: 8.1 ± 1.1%, Compound A low-dose: 8.0 ± 0.8%, Compound A high-dose: 8.5 ± 0.6%).

Whole blood glucose. Blood glucose levels significantly increased in the Disease-control group compared with the Normal group (Normal: 198 ± 30 mg/dL, Disease-control: 682 ± 63 mg/dL). Blood glucose levels significantly increased in the Telmisartan group compared with the Disease-control group (Telmisartan: 880 ± 39 mg/dL). There were no significant differences in blood glucose levels between the Vehicle group and the Compound A low-dose group (Vehicle: 605 ± 134 mg/dL, Compound A low-dose: 677 ± 36 mg/dL). Blood glucose levels tended to increase in the Compound A high-dose group compared with the Vehicle group (Compound A high-dose: 791 ± 54 mg/dL).

Serum ALT. Serum ALT levels tended to increase in the Disease-control group compared with the Normal group (Normal: 22 ± 4 U/L, Disease-control: 38 ± 8 U/L). There were no significant differences in serum ALT levels between the Disease-control group and the Telmisartan group or between the Vehicle group and either the Compound A low-dose or high-dose groups (Telmisartan: 35 ± 6 U/L, Vehicle: 58 ± 29 U/L, Compound A low-dose: 45 ± 17 U/L, Compound A high-dose: 49 ± 17 U/L).

Serum TG. Serum TG levels tended to increase in the Disease-control group compared with the Normal group (Normal: 137 ± 22 mg/dL, Disease-control: 558 ± 256 mg/dL). There was no significant difference in serum TG levels between the Disease control group and the Telmisartan group (Telmisartan: 843 ± 609 mg/dL). There was no significant difference in serum TG levels between the Vehicle group and the Compound A low-dose group (Vehicle: 391 ± 175 mg/dL, Compound A low-dose: 815 ± 754 mg/dL). Serum TG levels increased in the Compound A high-dose group compared with the Vehicle group (Compound A high-dose: 1145 ± 580 mg/dL).

Liver hydroxyproline content. Liver hydroxyproline content tended to increase in the Disease-control group compared with the Normal group (Normal: 0.74 ± 0.22 µg/mg, Disease-control: 1.09 ± 0.42 µg/mg). There were no significant differences in liver hydroxyproline content between the Disease-control group and the Telmisartan group or between the Vehicle group and either the Compound A low-dose or high-dose groups (Telmisartan: 0.95 ± 0.25 µg/mg, Vehicle: 0.79 ± 0.19 µg/mg, Compound A low-dose: 0.90 ± 0.26 µg/mg, Compound A high-dose: 0.95 ± 0.22 µg/mg).

**Liver TG content.** Liver TG content significantly increased in the Disease-control group compared with the Normal group (Normal: 8.8 ± 3.8 mg/g, Disease-control: 67.7 ± 17.7 mg/g). The Telmisartan group showed a significant decrease in liver TG content compared with the Disease-control group (Telmisartan: 30.6 ± 5.6 mg/g). There were no significant differences in liver TG content between the vehicle group and either the Compound A low-dose or high-dose groups (Vehicle: 58.8 ± 26.9 mg/g, Compound A low-dose: 64.8 ± 25.6 mg/g, Compound A high-dose: 67.8 ± 24.0 mg/g).

**HE staining and NAFLD Activity score.** Liver sections from the Disease-control group exhibited severe micro-and macrovesicular fat deposition, hepatocellular ballooning and inflammatory cell infiltration. Consistent with these observations, NAS significantly increased in the Disease-control group compared with the Normal group (Normal: 0.0 ± 0.0, Disease-control: 5.1 ± 1.1). Telmisartan group showed marked improvements in fat deposition, hepatocellular ballooning and inflammatory cell infiltration, with a significant reduction in NAS compared with the Disease-control group (Telmisartan: 2.6 ± 1.2). In the Compound A low-dose group, lobular inflammation was improved compared with the Vehicle group. No obvious difference was found in HE-stained sections between the Vehicle group and the Compound A high-dose group. NAS tended to decrease both in the Compound A low-dose and high-dose groups compared with the Vehicle group (Vehicle: 5.9 ± 1.0, Compound A low-dose: 4.8 ± 0.9, Compound A high-dose: 5.1 ± 0.6).

### Gene expression analyses.

**TIMP-1.** TIMP-1 mRNA expression levels were significantly up-regulated in the Disease-control group compared with the Normal group (Normal: 1.00 ± 0.25, Disease-control: 7.04 ± 2.13). TIMP-1 mRNA expression levels tended to be down-regulated in the Telmisartan group compared with the Disease-control group (Telmisartan: 4.10 ± 1.18). There were no significant differences in TIMP-1 mRNA expression levels between the Vehicle group and either the Compound A low-dose or high-dose groups (Vehicle: 7.74 ± 4.15, Compound A low-dose: 5.35 ± 2.82, Compound A high-dose: 6.73 ± 2.23).

**α-SMA.** Alpha-SMA mRNA expression levels tended to be up-regulated in the Disease-control group compared with the Normal group (Normal: 1.00 ± 0.60, Disease-control: 2.43 ± 1.33). There were no significant differences in α-SMA mRNA expression levels between the Disease-control group and the Telmisartan group (Telmisartan: 1.83 ± 0.36). Alpha-SMA mRNA expression levels tended to be down-regulated in the Compound A low-dose group compared with the Vehicle group (Vehicle: 3.19 ± 1.81, Compound A low-dose: 1.88 ± 0.60). There was no significant difference in α-SMA mRNA expression levels between the Vehicle group and the Compound A high-dose group (Compound A high-dose: 2.23 ± 1.23).

**Collagen Type 3.** Collagen Type 3 mRNA expression levels were significantly up-regulated in the Disease-control group compared with the Normal group (Normal: 1.00 ± 0.16, Disease-control: 2.27 ± 0.35). Collagen Type 3 mRNA expression levels tended to be down-regulated in the Telmisartan group compared with the Disease-control group (Telmisartan: 1.73 ± 0.33). Collagen Type 3 mRNA expression levels were significantly down-regulated in the Compound A low-dose group compared with the Vehicle group (Vehicle: 3.09 ± 1.51, Compound A low-dose: 1.93 ± 0.37). Collagen Type 3 mRNA expression levels tended to be down-regulated in the Compound A high-dose group compared with the Vehicle group (Compound A high-dose: 2.22 ± 0.46).

The Compound A low-dose group showed a significant decrease or decreasing trends in the expression levels of fibrosis-related genes (Collagen Type 3, TIMP-1 and α-SMA), while the Compound A high-dose group showed a significant reduction in the fibrosis area in liver histology. See, **Table 3.**

**TABLE 3. GENE EXPRESSION ANALYSES**

| | 12 weeks of age | | | 13 weeks of age | | |
|---|---|---|---|---|---|---|
| PARAMETER (mean ± SD) | NORMAL (n+8) | Disease-control (n=8) | Telmisartan (n=8) | Vehicle (n=8) | Compound A low-dose (n=8) | Compound A high dose (n=8) |
| | | | | | | |
| TIMP-1 | 1.00 ± 0.25 | 7.04 ± 2.13 | 4.10 ± 1.18 | 7.74 ± 4.15 | 5.35 ± 2.82 | 6.73 ± 2.23 |
| | | | | | | |
| α-SMA | 1.00 ± 0.60 | 2.43 ± 1.33 | 1.83 ± 0.36 | 3.19 ± 1.81 | 1.88 ± 0.60 | 2.23 ± 1.23 |
| | | | | | | |
| Collagen Type 3 | 1.00 ± 0.16 | 2.27 ± 0.35 | 1.73 ± 0.33 | 3.09 ± 1.51 | 1.93 ± 0.37 | 2.22 ± 0.46 |

### Example 4. SUMMARY AND DISCUSSION

These results show that Compound A has anti-fibrotic effects on the liver. The low-dose group also showed a reduction of lobular inflammation in the liver, with a decreasing trend in NAS. This indicates an anti-inflammatory effect of Compound A on steatohepatitis, in addition to the anti-fibrotic effects. Synergetic effects of these anti-fibrotic and anti-inflammatory activities can be particularly effective with administration of β-catenin inhibitors as represented by Compound A in a pro-fibrogenic phase of hepatic fibrosis.

## Claims

1. An alpha helix mimetic β-catenin inhibitor compound for use in the treatment of hepatic fibrosis, selected from:
4-(((6S,9S,9aS)-1-(benzylcarbamoyl)-2,9-dimethyl-4,7-dioxo-8-(quinolin-8-ylmethyl)octahydro-1H-pyrazino[2,1-c][1 ,2,4]triazin-6-yl)methyl)phenyl dihydrogen phosphate,
or (6S,9S,9aS)-N-benzyl-6-(4-hydroxybenzyl)-2,9-dimethyl-4,7-dioxo-8-(quinolin-8-ylmethyl)octahydro-1H-pyrazino[2,1-c][1,2,4]triazine-1-carboxamide,
wherein the hepatic fibrosis results from steatohepatitis.

2. The compound for use of claim 1, wherein the hepatic fibrosis results from non-alcoholic steatohepatitis (NASH).

3. A pharmaceutical composition comprising a compound as defined in claim 1 for use as defined in claim 1 or 2.

## Patentansprüche

1. Alpha-Helix-mimetische β-Catenin-Inhibitorverbindung zur Verwendung bei der Behandlung von Leberfibrose, ausgewählt aus:
4-(((6S,9S,9aS)-1-(benzylcarbamoyl)-2,9-dimethyl-4,7-dioxo-8-(chinolin-8-ylmethyl)octahydro-1H-pyrazino[2,1-c][1,2,4]triazin-6-yl)phenyldi-hydrogenphosphat oder
(6S,9S,9aS)-N-Benzyl-6-(4-hydroxybenzyl)-2,9-dimethyl-4,7-dioxo-8-(chinolin-8-ylmethyl)octahydro-1H-pyrazino[2,1-c][1,2,4]triazin-1-carboxamid,
wobei die Leberfibrose aus einer Steatohepatitis resultiert.

2. Verbindung zur Verwendung gemäß Anspruch 1, wobei die Leberfibrose aus einer nichtalkoholischen Steatohepatitis (NASH) resultiert.

3. Pharmazeutische Zusammensetzung, die eine Verbindung gemäß Anspruch 1 zur Verwendung gemäß Anspruch 1 oder 2 umfasst.

## Revendications

1. Composé inhibiteur de β-caténine mimétique d'hélice alpha pour une utilisation dans le traitement de la fibrose hépatique, choisi parmi :
le dihydrogénophosphate de 4-(((6S,9S,9aS)-1-(benzylcarbamoyl)-2,9-diméthyl-4,7-dioxo-8-(quinolin-8-ylméthyl)octahydro-1H-pyrazino[2,1-c][1,2,4]triazin-6-yl)méthyl)phényle, ou
le (6S,9S,9aS)-N-benzyl-6-(4-hydroxybenzyl)-2,9-diméthyl-4,7-dioxo-8-(quinolin-8-ylméthyl)octahydro-1H-pyrazino[2,1-c][1,2,4]triazine-1-carboxamide,
dans laquelle la fibrose hépatique résulte d'une stéatose hépatique.

2. Composé pour une utilisation selon la revendication 1, dans laquelle la fibrose hépatique résulte d'une stéatose hépatique non alcoolique (NASH).

3. Composition pharmaceutique comprenant un composé tel que défini dans la revendication 1 pour une utilisation telle que définie dans la revendication 1 ou 2.
